# EUROPEAN PATENT APPLICATION

(11) **EP 1 338 300 A1**
(43) Date of publication of application: **27.08.2003**
(21) Application number: 03290451.8
(22) Date of filing: 26.02.2003
(51) Int. Cl.: A61M 25/10

(54) **Balloon catheter**

(30) Priority: 26.02.2002 JP 2002049785; 29.01.2003 JP 2003020677
(71) Applicant: Tokai Kobunshi Kagaku Kabushikigaisha, Nagoya-shi, Aichi-ken (JP)
(72) Inventor: Miyata, Akira, Kumamoto-shi, Kumamoto-ken (JP); Tsutsui, Nobumasa, Nagoya-shi, Aichi-ken (JP); Tsutsui, Yasuhiro, Nagoya-shi, Aichi-ken (JP); Shinkai, Tomoyuki, Kasugai-shi, Aichi-ken (JP); Shizu, Yasunori, Kasugai-shi, Aichi-ken (JP)
(74) Representative: Intès, Didier Gérard André

(57) **Abstract**

A balloon catheter capable of expanding a narrowed site of a blood vessel in the vicinity of a curved part of the blood vessel, and of easily adapting the shape of the balloon to the shape of the curved part of the blood vessel. The balloon has a shape with a curved part, and the outer portion of the curved part (the shaded area) formed so as to have a smaller film thickness as compared with the remaining portion of the balloon is stretched by an amount larger than the remaining portion of the balloon. The difference in stretch amount between the outer portion of the curved part of the balloon and the remaining portion of the balloon becomes larger as the internal pressure of the balloon is increased. Accordingly, the extent of curve of the balloon can be controlled arbitrarily by controlling the internal pressure of the balloon.

## Description

### BACKGROUND OF THE INVENTION

### (1) Field of the Invention

The present invention relates to a balloon catheter.

### (2) Background Art

Conventional balloon catheters used in PTA (Percutaneous Transluminal Angioplasty) include a type of balloon catheter having a straight-shaped balloon when inflated and another type of balloon catheter having a slightly curved balloon when inflated.

By using one of these balloon catheters, angioplasty can be performed on a narrowed site of a straight or a slightly curved blood vessel.

However, there has been a problem that appropriate angioplasty cannot be performed using such a conventional balloon catheter when a narrowed site is within a largely curved part of a blood vessel.

For instance, a blood vessel in the shunt area of a patient being dialyzed, which is quite largely curved in many cases, often develops a stenosis (i.e. a narrowed site). When a straight-shaped balloon as mentioned above is to be inflated in such a curved part of the blood vessel, the balloon sometimes bends in the middle thereof or develops wrinkles or slack. Once the straight-shaped balloon has bent or developed wrinkles or slack, it is difficult to sufficiently inflate the balloon, with the result that appropriate angioplasty often cannot be performed on the narrowed site.

And it is worried that forcibly expanding the blood vessel in the shunt area using a straight-shaped balloon may cause an excessive deformation and stretch of the curved blood vessel, which could lead to damage of the blood vessel. Specifically, even if the whole balloon can be inflated, the balloon after being inflated has only a given shape and cannot be curved into a different shape. Accordingly, when how the balloon is curved is not exactly the same as how the blood vessel is curved, the top end of the balloon is pressed against the blood vessel wall as the balloon is inflated, which could lead to damage of the blood vessel wall, and furthermore, to undesirable stretch of the curved blood vessel.

An object of the present invention, which has been made to solve the above problems, is to provide a balloon catheter capable of expanding a narrowed site of a blood vessel in the vicinity of a curved part of the blood vessel, and of easily adapting the shape of the balloon to the shape of the curved part of the blood vessel.

### SUMMARY OF THE INVENTION

The above and other objects are attained by a balloon catheter which comprises a long shaft having an internal hollow and a balloon provided at the distal end of the shaft, the balloon being inflated/deflated in accordance with the pressure of the fluid supplied to the inside of the balloon through the internal hollow of the shaft, wherein the shape of the balloon when inflated has a curved part between the proximal end and the distal end of the balloon, and the extent of curve in the curved part becomes larger as the internal pressure of the balloon is increased.

The location of the curved part of the balloon is not restrictively limited, but preferably is closer to the distal end from the center of the balloon. The curved part is designed such that the maximum angle of the curved part when the maximum curve of the balloon is obtained matches the curve angle of the target blood vessel. For instance, the maximum angle of the curved part (i.e. the angle formed by the two balloon parts extending respectively toward both ends of the balloon from the curved part) may be almost 0 degree for a blood vessel which is bent as if folded back. On the contrary, the maximum angle of the curved part may be almost 180 degrees, for example 170 degrees, for a blood vessel which is only slightly curved.

According to the balloon catheter constituted as above, the shape of the balloon when inflated has a curved part between the proximal end and the distal end of the balloon, which allows the balloon to be inflated at a proper indwelling position even in a largely curved blood vessel without forcibly bending the balloon.

Also, since the balloon is designed such that the extent of curve in the curved part of the balloon becomes larger as the internal pressure of the balloon is increased, it is possible to change the curvature of the balloon so as to adapt the shape of the balloon to the shape of the curved part of the blood vessel by controlling the internal pressure of the balloon depending on how the blood vessel is curved.

Accordingly, it is possible to appropriately inflate the entire balloon instead of bending the balloon in the middle thereof or developing wrinkles or slack in the balloon, and thereby to perform appropriate angioplasty on a narrowed site. More particularly, unlike the case with a balloon having only a given shape after being inflated, it is possible to prevent the top end of the balloon from being pressed against the blood vessel wall by controlling the internal pressure of the balloon, thereby changing how to curve the balloon. Thus, damage to the blood vessel wall or undesirable stretch of the curved blood vessel can be avoided.

In the present balloon catheter, the balloon is designed such that a larger curve of the curved part of the balloon is obtained as the internal pressure is increased. In a particular example to achieve this, the outer portion of the curved part is made more stretchable as the internal pressure is increased than the inner portion of the curved part, and the difference in stretch amount between the outer portion and the inner portion of the curved part becomes larger as the internal pressure of the balloon is increased, whereby the extent of curve in the curved part becomes larger.

To make the outer portion of the curved part more stretchable as the internal pressure of the balloon is increased than the inner portion of the curved part, for instance, the film thickness of the outer portion of the curved part is made smaller than the film thickness of the inner portion.

It may be possible to form a balloon having an uneven film thickness from the beginning, or to form a balloon having an even film thickness by means of the primary process and make the film thickness uneven by means of the secondary process. The secondary process is, for instance, while softening a part of or the entire balloon with heat, stretching a part of the balloon under pressure, so that a partially thin balloon can be obtained. Alternatively, a partially thick balloon can be obtained by bonding or thermal welding a film on a part of the balloon.

Partially changing the thickness of the balloon is a mere means for making the outer portion of the curved part more stretchable as the internal pressure is increased than the inner portion of the curved part, but is not always necessary.

It is possible to form the outer portion of the curved part with a material more stretchable than the material of the inner portion of the curved part, so that the outer portion may be more stretchable as the internal pressure of the balloon is increased. In other words, even when the film thickness is even over the entire balloon, the outer portion and the inner portion of the curved part may be made different in stretchability by locally changing the material that forms the balloon, or by bonding or thermal welding two kinds of films different in elasticity to a part of the balloon. The balloon may be curved as the result of being inflated, regardless of whether the film thickness is even or not over the entire balloon, due to the fact that different materials are used for the outer and inner portions.

Preferable materials for forming the outer portion of the curved part are, for example, polyamide, polyethylene, polyurethane and thermoplastic elastomer. Preferable materials for forming the inner portion of the curved part are, for example, polyamide, polyethylene and polyethylene terephthalate. By combining the above materials, a balloon is formed to have film thicknesses of 20 to 40 µm in both the inner and outer portions of the curved part. The same kind of plastic (for example combining these materials) may be employed to obtain a curved balloon because the elasticity of the same kind of plastic can vary depending on the degree of crystallinity and orientation, while different kinds of plastics may also be employed.

Various kinds of materials different in stretchability, that is, some materials different in stretchability due to the difference in molecular structure and other materials different in stretchability due to the difference in degree of crystallinity may be arbitrarily employed. Alternatively, materials different in flexural modulus may be employed. For instance, when the outer portion of the curved part is made of a plastic material having a flexural modulus of 90-500 MPa and the inner portion of the curved part is made of a plastic material having a flexural modulus of 900-1500MPa, the difference in flexural modulus causes the balloon to be curved as the internal pressure is increased.

Furthermore, in the above described balloon catheter, a core material which is less stretchable but more flexible than the balloon may be provided inside the balloon, with the balloon fixed to the core material in the vicinity of both ends of the balloon.

This structure allows the core material to prevent the inner portion of the curved part from being stretched when the balloon is inflated.

In this case, if the core material is a tube having an internal hollow able to be used as a guidewire insertion lumen, and the balloon is fixed to the outer periphery surface of the tube in the vicinity of both ends of the balloon, insertion/pulling of the balloon catheter can be performed using a known guidewire.

As described above, according to a balloon catheter having the features of the present invention, it is possible to control the extent of curve by controlling the pressure of the fluid to be introduced into the balloon, so that a blood vessel is prevented from being curved excessively.

Also, the balloon can be designed so as to almost follow the original curve of the blood vessel by optimizing the properties of the material and the features of the structure of the balloon.

Further, in the balloon catheter according to the present invention, the balloon itself is curved to appropriately transmit the pressure to the blood vessel wall without deforming or stretching the curved blood vessel, and the top of the balloon stays inside the blood vessel due to its curve or a side surface of the top of the balloon merely contacts the blood vessel wall with planar (parallel) contact surfaces therebetween without damaging the blood vessel wall, unlike the case of expanding a curved blood vessel using a straight-shaped balloon by which the blood vessel is deformed or stretched linearly and, therefore, it is necessary to be careful to prevent damage to the blood vessel due to the

The balloon can be curved so that its extent of curvature matches the extent of curvature of the vessel. It is possible that, depending on the internal pressure increase, the balloon may be curved to a larger extent than the initial extent of curvature of the blood vessel, so that the balloon further curves the vessel. Even in this case, the top of the balloon stays inside the blood vessel due to its curve or a side surface of the top of the balloon merely contacts the blood vessel wall with planar (parallel) contact surfaces therebetween. Thus, somewhat excessive curve of the balloon would not lead to damage to the blood vessel wall or any clinical problems due to overly curving the blood vessel.

### BRIEF DESCRIPTION OF THE DRAWINGS

A preferred embodiment of the present invention will be described hereinafter with reference to the drawings, in which:
FIG. 1 is a side view of a balloon catheter according to the preferred embodiment of the invention;
FIGS. 2A to 2C are explanatory views illustrating the curve angle of the balloon in accordance with the internal pressure of the balloon; and
FIGS. 3A and 3B are explanatory views showing another example of the arrangement of an expandable portion and a less expandable portion of the balloon.

### DETAILED DESCRIPTION OF THE PREFERRED EMBODIMENT

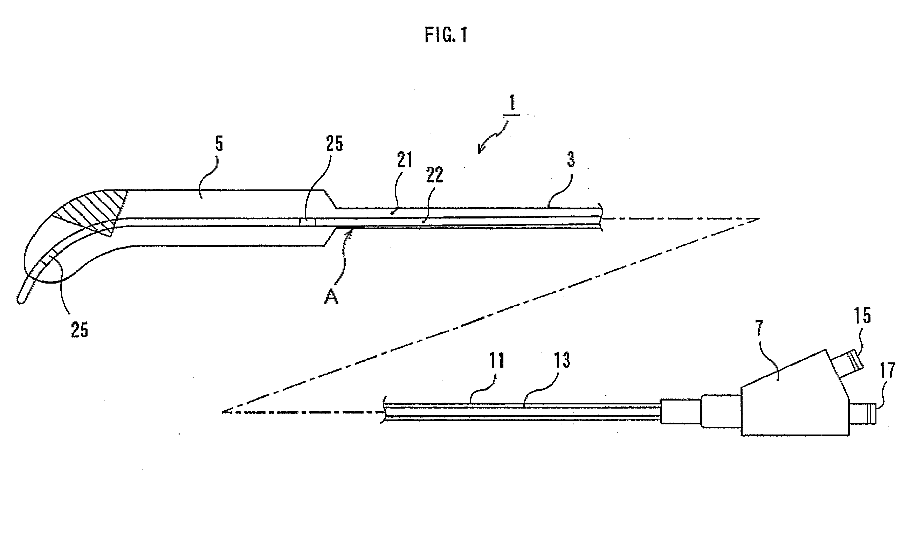

A balloon catheter 1 shown in Fig. 1 comprises a long shaft 3 with an internal hollow, a balloon 5 provided at the distal end of the shaft 3 and a connector 7 provided at the proximal end of the shaft 3.

The shaft 3 is a double pipe constituted by inserting an inner pipe 13 into the internal hollow of an outer pipe 11. The outer pipe 11 is a tube material made of polyamide, polyethylene, polyimide, polyetheretherketone, polyethylene terephthalate, polyurethane or polypropylene. The inner pipe 13 is a tube material made of polyamide, polyethylene, polyimide, polyetheretherketone, polyethylene terephthalate, polyurethane, polypropylene or fluoroplastic. The distal end of the outer pipe 11 is connected to the proximal end of the balloon 5, and the internal hollow of the outer tube 11 communicates with the inside of the balloon 5. The inner pipe 13 extends toward the distal direction further than the connection between the outer pipe 11 and the balloon 5, and passes through the inside of the balloon 5. The distal end of the balloon 5 is connected to the outer peripheral surface of the distal end of the inner pipe 13. In the vicinity of the connection between the outer pipe 11 and the balloon 5, the inner peripheral surface of the outer pipe 11 and the outer peripheral surface of the inner pipe 13 are connected (Point A in FIG. 1) with each other in order to prevent the outer pipe 11 and the inner pipe 13 from being relatively shifted in the axial direction.

The balloon 5 is a hollow body formed by a film of polyamide, polyethylene, polyurethane, thermoplastic elastomer or polyethylene terephthalate, and is inflated or deflated in accordance with the pressure of the fluid supplied to the inside of the balloon 5. As shown in FIG. 1, the balloon 5, when inflated, has a shape having a curved part between the proximal end and the distal end of the balloon 5. The curved part of the balloon 5 is positioned closer to the distal end from the center of the balloon 5, and the outer portion of the curved part (the shaded area in FIG. 1 in the present embodiment) is formed so as to have a smaller film thickness as compared with the remaining portion of the balloon 5 by means of heat treatment and pressure treatment.

The connector 7, which is a member used for connecting the balloon catheter 1 and a supply source of pressure fluid (not shown), is provided with a pressure fluid supply port 15 and a guidewire insertion port 17. Respective proximal ends of the outer pipe 11 and the inner pipe 13 are connected to the connector 7. The space (hereinafter also referred to as the "first lumen 21") between the inner periphery of the outer pipe 11 and the outer periphery of the inner pipe 13 communicates with the pressure fluid supply port 15 of the connector 7, and the internal hollow (hereinafter also referred to as the "second lumen 22") of the inner pipe 13 communicates with the guidewire insertion port 17 of the connector 7.

In the vicinity of the both ends of the balloon 5, metal markers 25 are mounted around the outer periphery of the inner pipe 13.

In the balloon catheter 1 constituted as above, when the pressure fluid is supplied through the pressure fluid supply port 15 of the connector 7, the pressure fluid is introduced to the inside of the balloon 5 through the internal hollow of the shaft 3 (the first lumen 21) and the balloon 5 is inflated, while when the pressure fluid inside the balloon 5 is drained, the balloon 5 is deflated.

When the balloon 5 is inflated, while the film forming the balloon 5 is somewhat stretched, the outer portion of the curved part of the balloon 5 (the shaded area in FIG. 1 in the present embodiment), which is formed so as to have a smaller film thickness as compared with the remaining portion of the balloon 5, is stretched by an amount larger than the remaining portion of the balloon 5. The difference in the stretch amount between the outer portion of the curved part of the balloon 5 and the remaining portion of the balloon becomes larger as the internal pressure of the balloon 5 is increased.

The inner pipe 13 disposed in the inside of the balloon 5 is flexible but less stretchable than the balloon 5, and the balloon 5 is fixed to the inner pipe 13 in the vicinity of the both ends of the balloon 5. Accordingly, the inner pipe 13 restrains the whole balloon 5 (particularly the inner portion of the curved part) from being stretched.

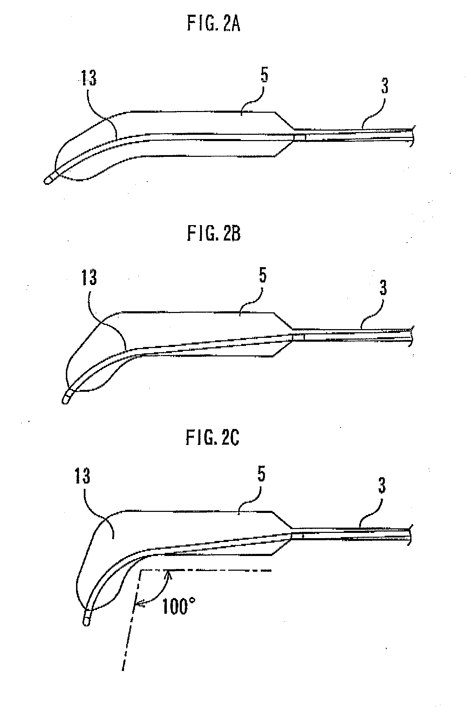

As a result, in the present balloon catheter 1, when the internal pressure of the balloon 5 is, for instance, approximately 5 atm., the balloon 5 is curved to the extent shown in FIG. 2A. When the internal pressure of the balloon 5 is increased up to approximately 10 atm., the balloon 5 is curved to the extent shown in FIG. 2B. When the internal pressure of the balloon 5 is further increased up to approximately 15 atm., the balloon 5 is curved to the extent shown in FIG. 2C. In other words, as the internal pressure of the balloon 5 is increased, the extent of curve in the curved part becomes larger. Therefore, the extent of curve of the balloon 5 can be controlled arbitrarily by controlling the internal pressure of the balloon 5. In this connection, the angle of the curved part of the balloon 5 becomes slightly less than 100 degrees when the maximum curve is obtained in the curved part (see FIG. 2C).

According to the balloon catheter 1 as described above, the shape of the balloon 5 when inflated has a curved part between the proximal end and the distal end of the balloon 5, which allows the balloon 5 to indwell at a proper indwelling position even in a largely curved blood vessel without forcibly bending the balloon 5.

Also, since the balloon 5 is designed such that the extent of curve in the curved part of the balloon 5 becomes larger as the internal pressure of the balloon 5 is increased, it is possible to change how to curve the balloon 5 thereby to adapt the shape of the balloon to the shape of the curved part of the blood vessel by controlling the internal pressure of the balloon 5 depending on how the blood vessel is curved.

Accordingly, it is possible to appropriately inflate the whole balloon 5 to perform appropriate angioplasty on a narrowed site. More particularly, unlike the case with a balloon having only a given shape after being inflated, it is possible to prevent the top end of the balloon 5 from being pressed against the blood vessel wall by controlling the internal pressure of the balloon 5 and thereby changing how to curve the balloon 5. Thus, damage to the blood vessel wall or undesirable stretch of the curved blood vessel can be avoided.

Although the present invention has been described with respect to a preferred embodiment, the present invention should not be limited to the embodiment and can be embodied in various forms.

For example, although the film thickness of the outer portion of the curved part is made smaller by means of heat treatment and pressure treatment so as to allow a larger curve of the curved part of the balloon 5 as the internal pressure is increased, another method for obtaining a curve in accordance with the internal pressure may be employed. Specifically, it may be possible that a balloon as a base has a small thickness so as to be relatively stretchable, and a less stretchable film is bonded or thermal welded to the inner portion of the curve part, so that the stretch amount can be different between in the inner portion and in the outer portion of the curved part when the internal pressure of the balloon is increased.

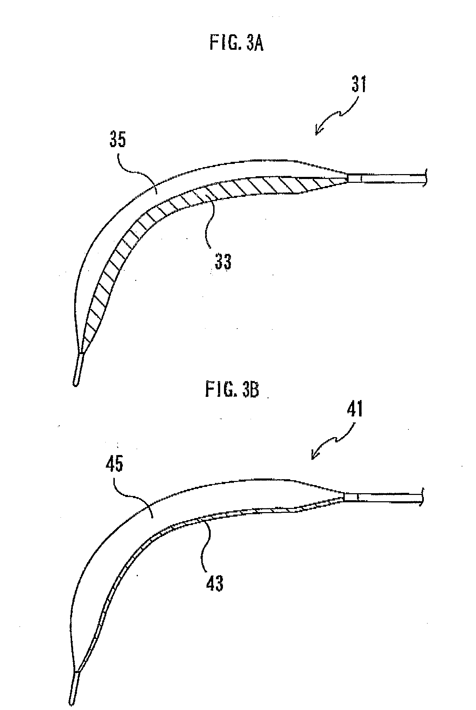

While the balloon 5 in the above described embodiment has a stretchable portion indicated by the shaded area in FIG. 1, the positional relation between a stretchable portion and a less stretchable portion may be appropriately adjusted depending on a variety of conditions, such as the extent of how the balloon should be curved and the pressure of the fluid to be introduced into the balloon. It may be possible, for instance, to employ a balloon 31 as shown in FIG. 3A in which a half part 33 (indicated by the shaded are in FIG. 3A) of the inner portion, when the balloon 31 is curved, is formed by a less stretchable film (e.g. a film of plastic material having a flexural modulus of 900-1500MPa), and the other half part 35 (indicated by the non-shaded are in FIG. 3A) is formed by a stretchable film (e.g. a film of plastic material having a flexural modulus of 90-500 MPa).

Alternatively, it may be possible to employ a balloon 41 as shown in FIG. 3B in which a region 43 (indicated by the shaded are in FIG. 3B) provided linearly as part of the balloon 41 is formed by a less stretchable material compared with the remaining region 45 (indicated by the non-shaded are in FIG. 3B).

## Claims

1. A balloon catheter comprising:
a long shaft (3) having an internal hollow (21); and
a balloon (5, 31, 41) provided at the distal end of the shaft,
the balloon being inflated/deflated in accordance with the pressure of the fluid supplied to the inside of the balloon through the internal hollow of the shaft,
**characterized in that** the shape of the balloon when inflated has a curved part between the proximal end and the distal end of the balloon, and the extent of curve in the curved part becomes larger as the internal pressure of the balloon is increased.

2. The balloon catheter according to claim 1, wherein the outer portion of the curved part is formed so as to be more stretchable as the internal pressure is increased than the inner portion of the curved part, and wherein the difference in stretch amount between the outer portion and the inner portion of the curved part becomes larger as the internal pressure of the balloon is increased, whereby the extent of curve in the curved part becomes larger.

3. The balloon catheter according to claim 2, wherein the outer portion of the curved part has a smaller film thickness than the inner portion of the curved part so as to be more stretchable as the internal pressure of the balloon is increased.

4. The balloon catheter according to claim 2, wherein the outer portion of the curved part is formed of a more stretchable material than the material of the inner portion of the curved part so as to be more stretchable as the internal pressure of the balloon is increased.

5. The balloon catheter according to claim 4, wherein the outer portion of the curved part is made of a plastic material having a flexural modulus of 90-500MPa and the inner portion of the curved part is made of a plastic material having a flexural modulus of 900-1500MPa.

6. The balloon catheter according to claim 6, wherein a core material which is less stretchable but more flexible than the balloon is provided inside the balloon, and wherein the balloon is fixed to the core material in the vicinity of both ends of the balloon.

7. The balloon catheter according to claim 6, wherein the core material comprises a tube provided with an internal hollow able to be used as a guidewire insertion lumen, and wherein the balloon is fixed to the outer periphery surface of the tube in the vicinity of both ends of the balloon.
